# EUROPEAN PATENT SPECIFICATION

(11) **EP 1 222 455 B1**
(45) Date of publication and mention of the grant of the patent: **21.06.2006**
(21) Application number: 00978901.7
(22) Date of filing: 04.10.2000
(51) Int. Cl.: G01N 27/327, C12Q 1/26, C12Q 1/32, C12Q 1/54

(54) **Sensor comprising an outer insulating layer comprising a plurality of electrically conductive fibers covered by a sensitive material on at least some of the fibres and voids between the fibres**
Sensor, bestehend aus einer isolierenden Ummantelung, enthaltend darin eine Vielzahl von leitfähigen Fasern, die zumindestens teilweise von einem sensitiven Material umgeben sind und Hohlräume zwischen den Fasern enthalten
Capteur comprenant une coche extérieure isolante, comprenant une pluralité des fibres électroconducteurs, au moins quelques sont couvrées par un materiau sensitive et des vides parmi les fibres

(30) Priority: 07.10.1999 US 414060; 05.11.1999 US 434254
(43) Date of publication of application: 17.07.2002
(73) Proprietor: Pepex Biomedical, LLC, Alameda, CA 94502 (US)
(72) Inventor: SAY, James, L., Alameda, CA 94502 (US); SAKSLUND, Henning, Pleasant Hill, CA 94523 (US); TOMASCO, Michael, F., Danville, CA 94526 (US)
(74) Representative: Leifert & Steffan
(86) International application number: PCT/US2000/041068
(87) International publication number: WO 2001/025474

(56) References cited:
- EP-A- 0 592 805
- EP-A- 0 710 835
- EP-A- 0 792 620
- WO-A-96/06947
- WO-A-97/15827
- US-A- 4 908 115
- US-A- 4 945 896

## Description

### Field of the Invention

This invention relates to sensors for measuring bioanalytes and to methods for making such sensors. More particularly, the invention relates to sensors for sensing lactate and to methods for making such sensors.

### Background of the Invention

Lactate is a small molecule that is produced by all tissues and organs of a patient's body that are in "distress." Wherever in the patient's body the demands for oxygen exceed the supply, then a state of low perfusion exists and lactate is produced. For example, lactate is produced if a patient is bleeding, if a patient's heart is failing, if a person's limb is in danger of being lost, or if a person is not getting enough oxygen to breathe. Thus many life and limb threatening clinical states produce elevated blood lactate levels, even in the face of adequate oxygen delivery to the patient. It is a matter of oxygen supply and metabolic demand.

At the cellular level, lactate is inversely proportional to the vital cellular energy stores of adenosine triphosphate and is produced within six seconds of inadequate perfusion or cellular injury. It is thus an ideal biochemical monitor of cellular viability at the tissue level, and of patient viability at the systemic level.

Clinically, the dire significance of elevated and rising blood lactate values is known. Trauma physicians and clinical evidence support the hypothesis that a simple, inexpensive, continuous, monitor of lactate in the trauma setting, will save lives by providing timely, life-saving information that will help dictate triage and therapy. For example, an emergency room patient who has a blood lactate level of 4mM has a 92% mortality rate within the next 24 hours. If this level is 6mM, then the mortality rate rises to 98%. In animal experiments, blood lactate levels begin to rise within minutes of hemorrhage, and conversely, begin to fall just as quickly with adequate resuscitation. In multivariate analysis, blood lactate is the best indicator of the degree of shock (superior to blood pressure, heart rate, urine output, base deficit, blood gas and Swan-Ganz data) and is proportional to the shed blood volume. Blood lactate levels correlate with a trauma patient's chances of survival. Therapy that fails to control a patient's increasing lactate levels must be modified or additional diagnoses quickly sought.

Sensors have been developed for detecting lactate concentrations in a given fluid sample. For example, United States Patent Nos. 5,264,105; 5,356,786; 5,262,035; and 5,320,725 disclose wired enzyme sensors for detecting analytes such as lactate or glucose. EP-A592805 discloses a sensor electrode comprising a plurality of carbon fibres that are dipped in a solution containing a reactant. The carbon electrode is impregnated with the solution and is covered with an insulating outer layer. Because the reactant is liquid no voids are defined between the covered fibres.

### Summary of the Invention

One aspect of the present invention relates to a sensor including a plurality of electrically conductive fibers. The sensor also includes a sensing material coating at least some of the fibers, and an insulating layer positioned about the electrically conductive fibers. The conductive fibers provide a large substrate surface area for supporting the sensing material. Thus, the sensor has a large surface area of sensing material even at small sizes. This large surface area of sensing material provides numerous advantages. For example, the large surface area assists in improving the response/sensing time of the sensor. Also, the large surface area assists in lengthening the useful life of the sensor.

Another aspect of the present invention relates to a surgical retractor device including a surgical retractor blade, and a lactate sensor positioned adjacent to the retractor blade for sensing lactate levels in tissue being compressed by the retractor blade. The lactate sensor allows a surgeon to monitor and detect when tissue being compressed by the retractor blade begins to become stressed.

These and various other features which characterize the invention are pointed out with particularity in the attached claims. For a better understanding of the invention, it's advantages, and objectives obtained by its use, reference should be made to the drawings and to the accompanying description, in which there is illustrated and described preferred aspects of the present invention.

### Brief Description of the Drawings

The accompanying drawings, which are incorporated in and constitute a part of this specification, illustrate several aspects of the invention and together with the description, serve to explain the principles of the invention. A brief description of the drawings is as follows:
Fig. 1 is an elevational view of a sensor constructed in accordance with the principles of the present invention;
Fig. 2 is a cross-sectional view taken along section line 2-2 of Fig. 1 with only a portion of the fiber ends depicted;
Fig. 3 is a detailed end view of one of the fibers of the sensor of Fig. 1, the fiber is coated with a sensing material;
Fig. 4 is a elevational view of an alternative sensor constructed in accordance with the principles of the present invention;
Fig. 5 is a schematic view of a sensor system incorporating the sensor of Fig. 1;
Fig. 6A is a schematic view of a sensor assembly constructed in accordance with the principles of the present invention, the sensor assembly is shown in a start or calibration condition;
Fig. 6B illustrates the sensor assembly of Fig. 6A in a test condition;
Fig. 6C illustrates the sensor assembly of Fig. 6A in a purge condition;
Fig. 7 is an exploded, perspective view of an alternative sensor assembly constructed in accordance with the principles of the present invention;
Fig. 8 is a longitudinal cross-section view of the assembled sensor assembly of Fig. 7;
Fig. 9 is a schematic illustration of a method for manufacturing a sensor such as the sensor of Fig. 1;
Fig. 10 illustrates another sensor system constructed in accordance with the principles of the present invention;
Fig. 11 is a partial left-end view of the sensor of Fig. 10;
Fig. 12 is a plan view of a further sensor constructed in accordance with the principles of the present invention;
Fig. 13 is an additional sensor constructed in accordance with the principles of the present invention; and
Fig. 14 schematically illustrates a system for the lactate level in tissue that is compressed by a surgical retractor.

### Detailed Description

Reference will now be made in detail to exemplary aspects of the present invention which are illustrated in the accompanying drawings. Wherever possible, the same reference numbers will be used throughout the drawings to refer to the same or like parts.

An aspect of the present invention relates to sensors for providing on-line monitoring/measurement of bioanalytes in a patient. One particular aspect of the present invention relates to a sensor for providing on-line measurement of lactate concentrations in a patient.

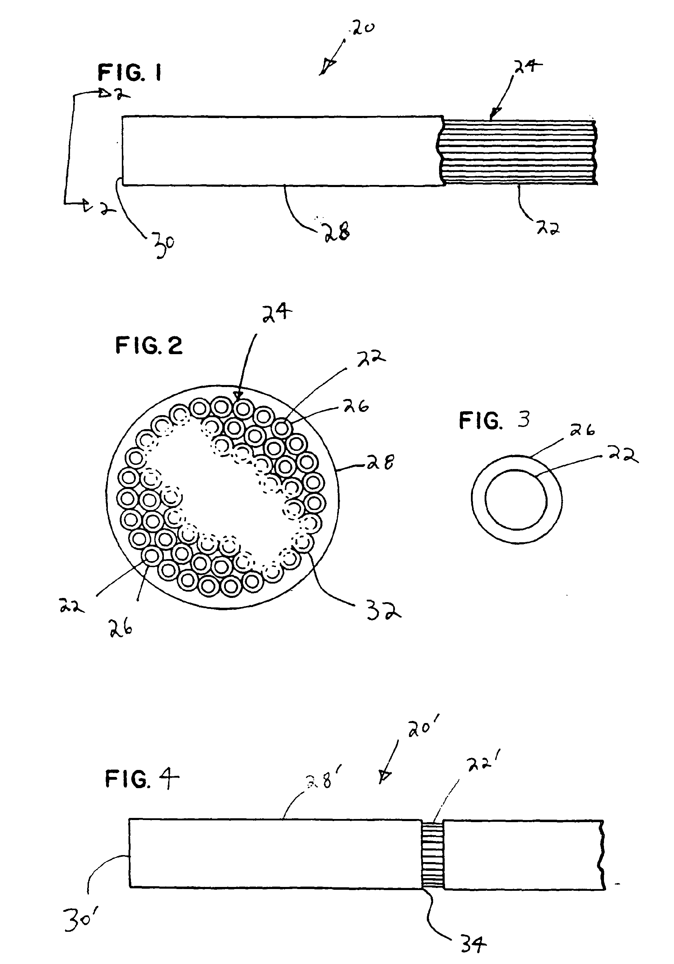

Figs. 1-3 illustrate a sensor 20 constructed in accordance with the principles of the present invention. The sensor 20 includes a plurality of electrically conductive fibers 22 arranged in a bundle 24. The fibers 22 in the bundle 24 are coated (i.e. covered) with a sensing material 26. An insulating layer 28 surrounds the bundle 24.

The fibers 22 of the sensor 20 are made of an electrically conductive material. A preferred material of the fibers 22 is carbon. For example, in one nonlimiting embodiment of the present invention, the fibers 22 are made of 92-98% carbon. The fibers 22 will each typically have a relatively small diameter. For example, in one particular nonlimiting environment, the fibers 22 can each have a diameter in the range of 5-10 microns. It will be appreciated that the illustrated embodiments are not drawn to scale. While any number of fibers 22 could be used to form the bundle 24, it is preferred for many fibers (e.g., 1,000 to 3,000 fibers per bundle) to be used. Preferably, the bundle 24 has a diameter in the range of .010 - .015 inches.

The sensing material 26 preferably includes a redox compound or mediator. The term redox compound is used herein to mean a compound that can be oxidized or reduced. Exemplary redox compounds include transition metal complexes with organic ligands. Preferred redox compounds/mediators are osmium transition metal complexes with one or more ligands having a nitrogen containing heterocycle such as 2, 2'-bipyridine. The sensing material can also include a redox enzyme. A redox enzyme is an enzyme that catalyzes an oxidation or reduction of an analyte. For example, a glucose oxidase or glucose dehydrogenase can be used when the analyte is glucose. Also, a lactate oxidase or lactate dehydrogenase fills this role when the analyte is lactate. In systems such as the one being described, these enzymes catalyze the electrolysis of an analyte by transferring electrons between the analyte and the electrode via the redox compound.

The insulating layer 28 of the sensor 20 preferably serves numerous functions to the sensor 20. For example, the insulating layer 28 preferably electrically insulates the fibers 22. Additionally, the insulating layer 28 preferably provides mechanical strength for maintaining the fibers 22 in the bundle 24. Additionally, the insulating layer 28 preferably forms a barrier about the fibers 22 that prevents the uncontrolled transport of a substance desired to be sensed (e.g., an analyte such as glucose or lactate). In one nonlimiting embodiment, the insulating layer 28 is made of a polymeric material such as polyurethane.

The insulating layer 28 preferably defines an opening for allowing a substance desired to be sensed to be transported or otherwise conveyed to the sensing material 26. For example, the sensor 20 can include a distal end 30 that is transversely cut. At the distal end 30, the insulating layer 28 defines an opening 32 (shown in Fig. 2) through which the material desired to be sensed can be transported. For example, the opening 32 is configured to allow an analyte such as lactate or glucose to diffuse into the sensing material 26 that surrounds the fibers 22.

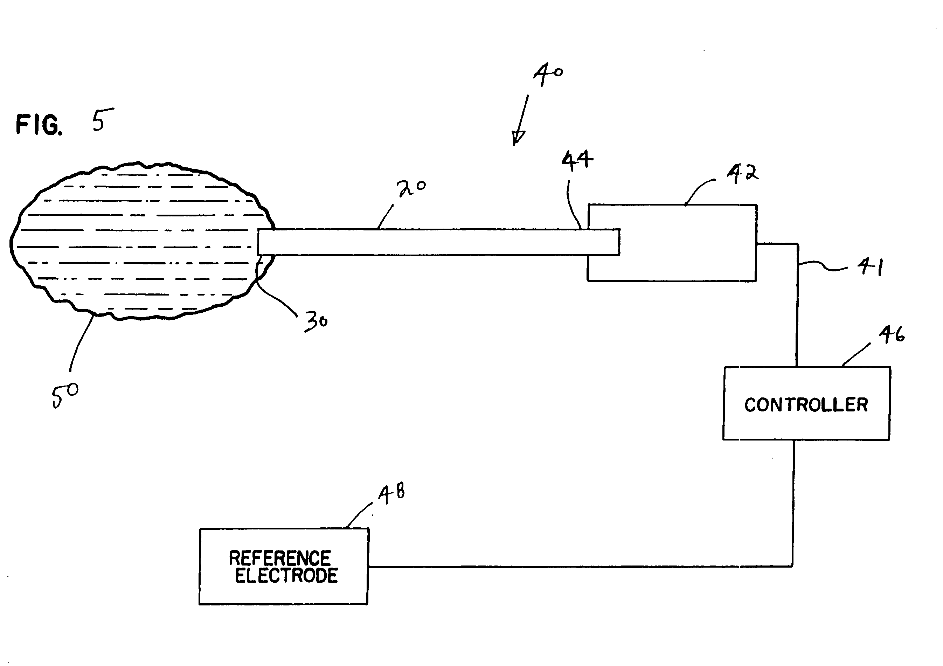

It will be appreciated that openings can be formed at various locations along the length of the sensor 20. For example, Fig. 4 illustrates an alternative sensor 20' having an opening 34 formed at an intermediate location along the length of the sensor 20'. The opening 34 is arranged in the form of an annular cut form through an insulating layer 28' of the sensor 20'. Fibers 22' coated with sensing material are located within the insulating layer 28'. The opening 34 exposes a region of the sensing material to the exterior of the sensor 20'. Consequently, the opening 34 provides a passage for allowing a substance desired to be sensed to diffuse into the region of exposed sensing material. The sensor 20' preferably also includes a distal end 30' that is closed or otherwise sealed by the insulating layer 28'.

Fig. 5 illustrates a sensing system 40 that incorporates the sensor 20 of Figures 1-3. The fibers 22 are electrically connected to a wire 41 by one or more electrical connectors 42 positioned at a proximal end 44 of the sensor 20. The wire 41 electrically connects the sensor 20 to a controller 46. The controller 46 can be any type of controller such as a micro-controller, a mechanical controller, a software driven controller, a hardware driven controller, a firmware driven controller, etc. The controller 46 is also electrically connected to a reference electrode 48. The reference electrode 48 preferably includes a layer of silver silver-chloride.

In use of the sensing system 40, the distal end 30 of the sensor 20 is placed in fluid communication with a test volume 50 of a substance containing an analyte desired to be sensed. The test volume 50 is the volume from which the analyte desired to be sensed can diffuse into the sensor 20 during the sensing period. With the sensor 20 so positioned, the analyte within the test volume 50 can diffuse into the sensing material 26 located adjacent to the distal end 30 of the sensor 20. Additionally, water within the test volume 50 can diffuse into the sensing material 26 such that the sensing material 26 is hydrated. A potential is then applied between the reference electrode 48 and the sensor 20. When the potential is applied, an electrical current will flow through the test volume 50 between the reference electrode 48 and the distal end 30 of the sensor 20. The current is a result of the electrolysis of the analyte in the test volume 50. This electrochemical reaction occurs via the redox compound in the sensing material 26 and the optional redox enzyme in the sensing material 26. By measuring the current flow generated at a given potential, the concentration of a given analyte in the test sample can be determined. Those skilled in the art will recognize that current measurements can be obtained by a variety of techniques including, among other things, coulometric, potentiometric, amperometric, voltammetric, and other electrochemical techniques.

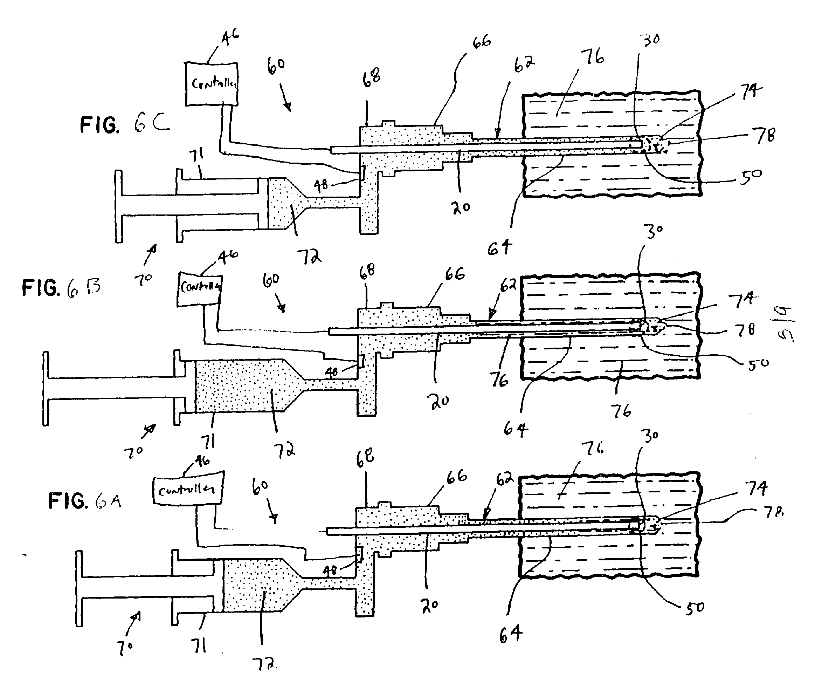

Figs. 6A-6C schematically illustrate a sensor assembly 60 for providing on-line monitoring/measurement of bioanalytes such as lactate in a patient. The sensor assembly 60 includes a catheter 62 (e.g., a peripheral catheter) having a catheter sheath 64 connected to a catheter hub 66 (i.e., a luer fitting). The sensor assembly 60 also includes an adapter 68 connected to the catheter hub 66. The adapter is in fluid communication with a pump 70 (e.g., a syringe 71 driven by a syringe driver (not shown)). The syringe 71 preferably contains a volume of calibration fluid 72.

The sensor assembly 60 of Fig. 5 is preferably incorporated into the sensor assembly 60. For example, as shown in Figs. 6A-6C, the sensor 20 extends through the adapter 68 and into the catheter sheath 64 such that the distal end 30 of the sensor 20 is located adjacent a tip 74 of the catheter sheath 64. In certain embodiments, a radial spacing of at least .0015 inches exists between the outer surface of the sensor 20 and the inner surface of the sheath 64. Also, the reference electrode 48 is shown positioned within the adapter 68 and both the reference electrode 48 and the sensor 20 are shown electrically connected to the controller 46.

As indicated above, the syringe 71 preferably contains a calibration fluid 72. The calibration fluid 72 preferably includes a predetermined concentration of a calibrant such as lactate or lactate sensors or glucose for glucose sensors. The calibration fluid can include a variety of other components in addition to a calibrant. For example, an anticoagulant such as sodium citrate can be used. A preferred calibration fluid comprises a solution of sodium citrate, saline, and lactate. Of course, lactate is only used as a calibrate if a lactate sensor is being used in the system. Other types of calibrates that may be used in the system include glucose, potassium, sodium, calcium, and ringers lactate.

Fig. 6A illustrates the sensor assembly 60 at a start condition. As shown in Fig. 6A, the catheter sheath 64 is inserted within a patient such that blood 76 surrounds the tip 74 of the catheter sheath 64. At the start condition, the catheter sheath 64 is filled with the calibration fluid 72 such that the distal tip 30 of the sensor 20 is bathed in the calibration fluid 72. It will be appreciated that with the catheter sheath 64 inserted within the patient, a diffusion zone 78 exists adjacent the catheter sheath tip 74. The diffusion zone 78 is the region into which blood can readily diffuse or mix even when the system is static.

Still referring to Fig. 6A, the test volume 50 of the sensing system 40 surrounds the distal end 30 of the sensor 20. The test volume 50 includes the volume surrounding the distal end 30 of the sensor 20 that is readily depleted of a test substance (e.g., lactate or glucose) when potential is applied between the sensor 20 and the reference electrode 48. It is preferred for the test volume 50 to not be coextensive with the diffusion zone 78. To achieve this, it is preferred for the distal end 30 of the sensor 20 to be located at least one-half millimeter away from the tip 74 of the catheter sheath 64. In certain embodiments, the distal end 30 of the sensor 20 is located in the range of 2 to 3 millimeters away from the catheter sheath tip 74.

While the reference electrode 48 is shown positioned within the adapter 68, it will be appreciated that other configurations could also be used. For example, the reference electrode 48 could comprise a skin mounted electrode positioned on the patient's skin adjacent to the catheter sheath 64. Furthermore, as shown herein, only two electrodes (i.e., the reference electrode 48 and the sensor 20) are used in the sensor assembly 60. It will be appreciated that in alternative embodiments, three electrodes (e.g., a reference electrode, a counter electrode, and a worker electrode) can be used. Exemplary wired enzyme sensors having three electrode configurations are described in United States Patent Nos. 5,264,105; 5,356,786; 5,262,035; and 5,320,725, which are hereby incorporated by reference.

Referring again to Fig. 6A, with the distal end 30 of the sensor 20 bathed in the calibration fluid, a potential can be applied between the reference electrode 48 and the sensor 20. When the potential is applied between the sensor 20 and the reference electrode 48, the sensing material 26 begins to consume the sensed analyte (i.e., the analyte desired to be sensed or measured such as lactate or glucose) within the calibration fluid located in the test volume 50. Initial calibration can be obtained by monitoring the slope of decay in the current generated between the sensor 20 and the reference electrode 48. A reading is preferably taken when the sensor 20 begins to consume all of the analyte in the test volume 50 and the current begins to decline.

After the sensor 20 has been calibrated, a blood sample can be tested. For example, as shown in Fig. 6B, to test a blood sample, the syringe plunger is drawn back such that blood 76 is drawn into the catheter sheath 64. Preferably, sufficient blood 76 is drawn into the catheter sheath 64 to surround the distal end 30 of the sensor 20 with blood and to ensure that the test volume 50 is filled with blood. Once sufficient blood has been drawn into the catheter sheath 64, movement of the plunger is stopped and a potential is applied between the sensor 20 and the reference electrode 48. With the potential applied between the reference electrode 48 and the sensor 20, the sensor 20 begins to consume the sensed analyte contained within the blood 76 within the test volume 50. When the sensor 20 approaches consuming all of the analyte within the test volume 50, the current begins to decline and a reading is taken.

Thereafter, the system is purged as shown in Fig. 6C by pushing the plunger of the syringe 71 inward causing the calibration fluid to displace the blood 76 within the sheath 64. Consequently, the blood 76 within the sheath 64 is forced back into the patient. Preferably, the syringe 71 injects enough of the calibration fluid 72 into the system to displace about two times the volume of the catheter sheath 64. As a result, some of the calibration fluid is injected into the patient along with the blood 76.

After the system has been purged, the sensor 20 can be recalibrated as described with respect to Fig. 6A. Thereafter, the testing and purging steps can be repeated.

The sensor 20 provides numerous advantages. For example, the plurality of fibers 22 provide a large surface area for supporting the sensing material 26. Therefore, a large surface area of sensing material 26 is exposed to the test volume 50. As a result, the sensor 20 is capable of quickly depleting the sensed analyte within the test volume 50 thereby allowing an analyte concentration to be quickly determined. This rapid sensing capability is particularly advantageous for applications such as fetal monitors and intercranial monitors. The large surface area also prevents the sensing material 26 from quickly becoming depleted thereby lengthening the useful life of the sensor 20. Furthermore, the use of carbon fibers assists in accurately calibrating the sensor 20 because carbon is an effective heat conductor. This is significant because some calibration processes are temperature dependent. By using a heat conductive fiber, the temperature of the fiber will quickly match the temperature of a calibration fluid contained within the test volume 50. As a result, calibration inaccuracies associated with differences in temperature between the calibration fluid and the sensor 20 can be reduced.

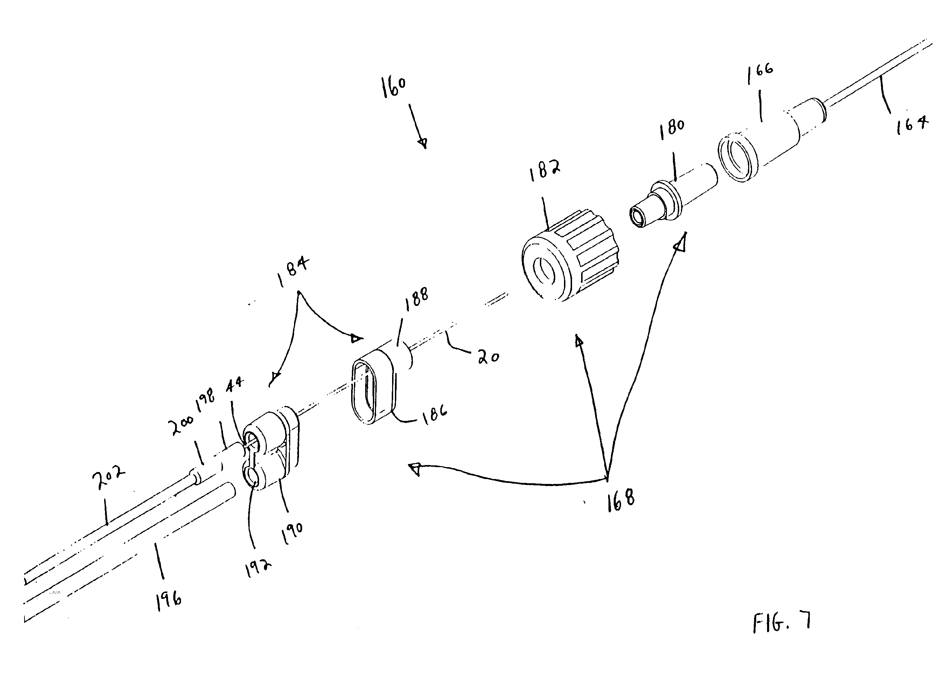

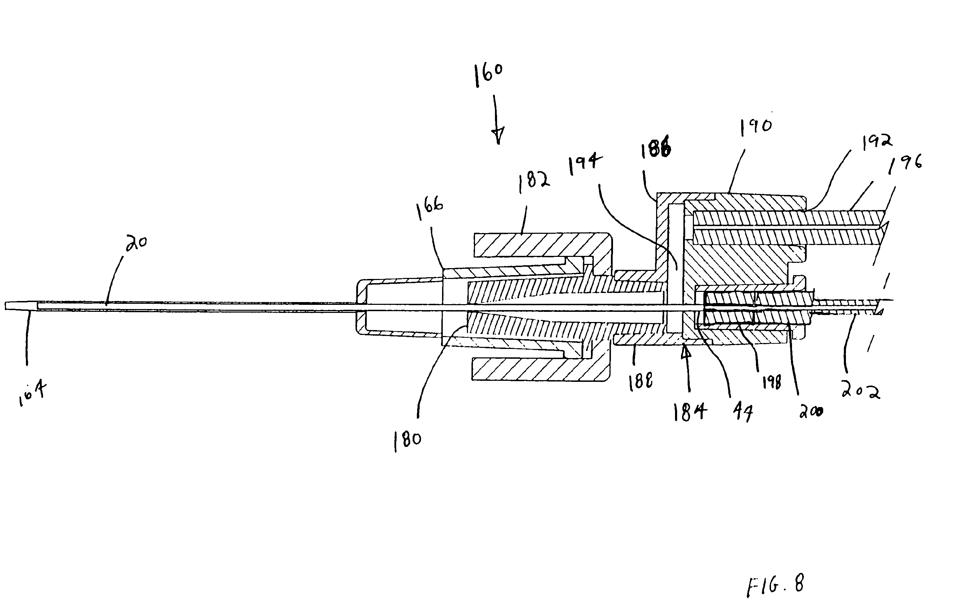

Figures 7 and 8 illustrate an alternative sensor assembly 160 constructed in accordance with the principles of the present invention. The sensor assembly 160 includes an adapter 168 that connects to a luer fitting 166 of a catheter sheath 164. The adapter 168 includes an insertion piece 180 that fits within the luer fitting 166, and a cap 182 that threads on the luer fitting 166 to hold the insertion piece 180 within the luer fitting 166. The adapter 168 also includes a two-piece manifold 184. The manifold 184 includes a first piece 186 having a projection 188 that extends through the cap 182 and provides a fluid-tight connection with the insertion piece 180. The manifold 182 also includes a second piece 190 that connects with the first piece 186. The second piece 190 includes a tube receiver 192. The first and second pieces 186 and 190 of the manifold 184 cooperate to define a flow passageway 194 (shown in Fig. 8) that extends from the tube receiver 192 to the insertion portion 180 of the adapter 168. In use, the tube receiver 192 preferably receives a tube 196 coupled to a source of calibration fluid (e.g., a syringe containing calibration fluid such as the syringe 71 of Figs. 6A-6C).

Still referring to Figures 7 and 8, the sensor 20 preferably extends through the adapter 168 and into the catheter sheath 164. A first electrical connector 198 is mounted at the proximal end 44 of the sensor 20. The first electrical connector 198 is electrically coupled to a second electrical connector 200 that is mounted at the end of a wire 202. Preferably, the wire 202 is electrically coupled a controller such as the controller 42 of Fig. 4.

It is noted that the sensor assembly 160 does not include an internal reference electrode. Instead, the sensor assembly 160 can include an external reference electrode (e.g., a skin-mounted electrode) that is coupled to the controller.

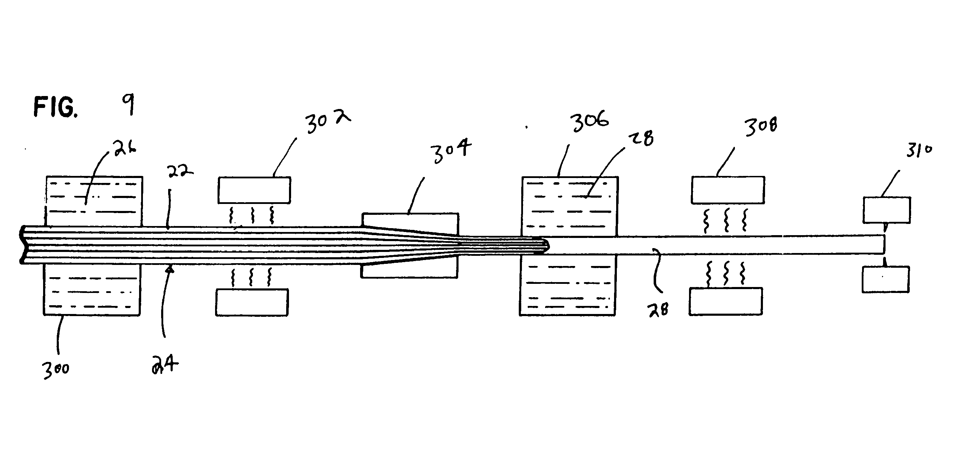

Figure 9 illustrates a method for making the sensor 20 of Figures 1-3. In practicing the method, the bundle 24 of fibers 22 is first pulled through a die 300 containing a volume of the sensing material 26 in liquid form. As the bundle 24 is pulled through the die 300, the sensing material 26 coats the outer surfaces of the fibers 22.

After the sensing material 26 has been applied to the fibers 22, the sensing material 26 can be dried at a heating station 302 (e.g., a convection heater). Thereafter, the fibers 22 coated with sensing material 26 are pulled through a sizing die 304 to compress the bundle 24 to a desired diameter. Next, the sized bundle 24 is pulled through a die 306 containing material that will form the insulating layer 28 of the sensor 20. For example, the die 306 can contain a volume of liquid polymer such as polyurethane. As the bundle 24 is pulled through the die 306, the insulating layer material coats the outside of the bundle. After the insulating layer 28 has been coated around the exterior of the bundle 24, the bundle can be moved through a curing station 308 (e.g., an ultraviolet curing station) where the insulating layer 28 is cured. Finally, the bundle 24 is moved through a cutting station 310 where the bundle 24 is cut into pieces having desired lengths.

The above-described method provides numerous advantages. For example, the method allows a relatively large number of sensors 20 to be manufactured in a relatively short amount of time. Also, the above-described method is able to provide sensors having similar operating characteristics from batch to batch.

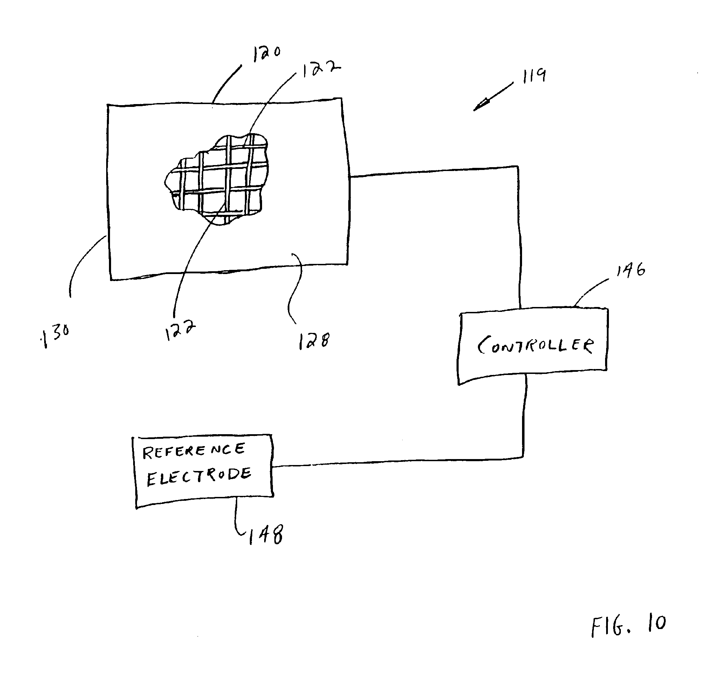

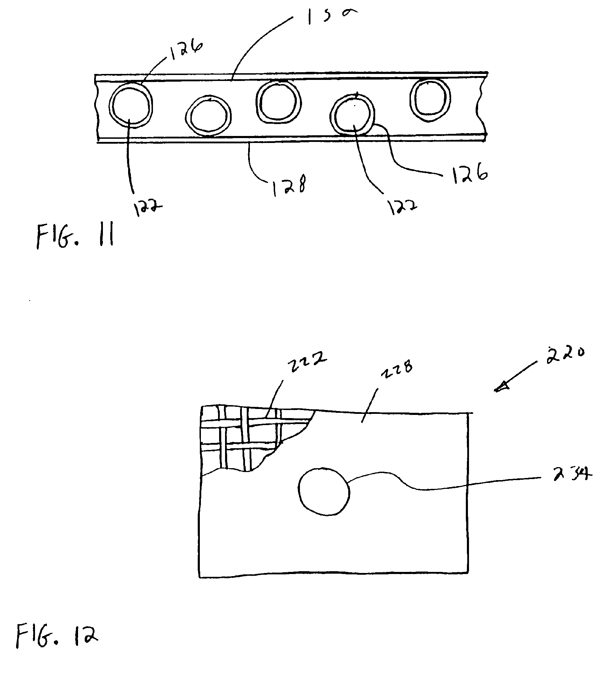

Fig. 10 illustrates a sensor system 119 constructed in accordance with the principles of the present invention. The sensor system 119 includes a sensor 120 having a plurality of electrically conductive fibers 122. As best shown in Fig. 11, the fibers 122 are coated (i.e., covered or encased) with a sensing material 126. An insulating layer 128 (e.g., a sheath) surrounds or encloses the plurality of fibers 122 to form an exterior boundary about the fibers 122.

The fibers 122 of the sensor 120 are arranged in a sheet-like configuration. For example, as shown in Fig. 10, the fibers 122 form a fabric having a square weave-type mesh. However, in alternative embodiments, the sheet can be formed by a matte of randomly arranged fibers, or the fibers can be arranged in a weave or pattern. The fibers 122 are electrically conductive. For example, in one embodiment, the fibers 122 are made of carbonized nylon.

In one non-limiting embodiment of the present invention, the fibers are arranged in the form of a carbonized nylon fabric. One exemplary type of fabric is sold by Sefar America, Inc. under the trade name "Carbotex" (e.g., product numbers C382/137 and C3130/49). These particular illustrative fabrics include monofilament fibers approximately 45 microns in diameter. These non-limiting fabrics also include square weaves with pore sizes of 130 microns and 82 microns respectively, and a thickness of about 92 microns. Preferably, the monofilament surfaces are evenly carbonized to a depth of a few microns with minimal discontinuities, making the surface particularly suitable for forming the substrate for wired enzyme biosensors. Preferably, the fibers have diameters less than 90 microns. More preferably, the fibers have diameters less than 60 microns. Most preferably, the fibers have diameters no greater than 45 microns.

Referring back to Fig. 10, the sensor 120 is electrically connected to a controller 146. The controller 146 is also electrically connected to a reference electrode 148. The controller 146 and the reference electrode 148 preferably operate in a manner similar to the reference electrode and controller previously described with respect to the embodiment of Fig. 5.

The insulating layer 128 of the sensor 120 performs the same function as the insulating layer 28 previously described with respect to the sensor 20 of Figs. 1-3. Similarly, the sensing layer 126 of the sensor 120 preferably performs these same function as the sensing layer 126 of the sensor 20 of Figs. 1-3. Accordingly, the descriptions pertaining to the insulating layer 28 and the sensing material 26 also apply to the insulating layer 128 and the sensing material 126.

The insulating layer 128 preferably defines an opening for allowing a substance desired to be sensed to be transported or otherwise conveyed to the sensing material 126. For example, the sensor 120 can have a distal end 130 that is transversely cut. At the distal end 130, the insulating layer 128 defines an opening 132 (shown in Fig. 11) through which the substance desired to be sensed can be transported. For example, the opening 132 is configured to allow an analyte such as lactate or glucose to diffuse into the sensing material 126 that surrounds the fibers 122.

It will be appreciated that alternative sensors can have access openings located at a variety of different locations. For example, Fig. 12 illustrates an alternative sensor 220 having an opening 234 that is transversely cut entirely through a mid-region of the sensor 220. Preferably, an outer boundary of the sensor 220 is sealed. Electrically conductive fibers 222 coated with sensing material are enclosed within an insulating sheath 228. The opening 234 through the sheath 228 allows regions of the fibers 222 adjacent to the middle of the sensor 220 to be exposed to a fluid containing an analyte desired to be sensed.

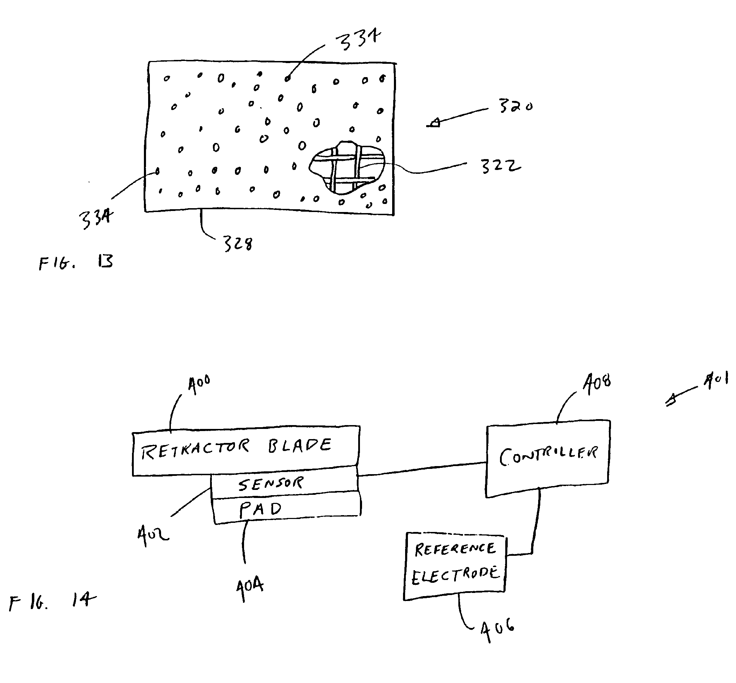

Fig. 13 illustrates a further sensor 320 constructed in accordance with the principles of the present invention. A sensor 320 includes a plurality of electrically conductive fibers 322 located within an insulating sheath 328. The fibers 322 are preferably covered with a sensing material. The sensing material is exposed to fluid containing an analyte desired to be sensed by a plurality of openings 334 defined though the insulating sheath 328.

To fabricate the sensor of Figs. 10 and 11, a sensing chemistry is deposited on a region of a mesh of electrically conductive fibers such that the mesh within the region is preferably substantially evenly coated on all aspects. Next, the mesh can be laminated to a carrier film such as a mylar or other suitable substrate using an elastomeric/adhesive layer that bonds to the substrate and encapsulates the mesh. Preferably, at least one area of the mesh is left exposed at some location remote from that which is covered with the sensing chemistry to provide an electrical contact surface for the sensor output. The sensor profile (i.e., the outer shape of the sensor) is then slit or otherwise cut (e.g., die cut) from the fabric in such a manner as to have at least one cutting plain that cuts through the portion of the mesh that is coated with sensing chemistry. The resultant cut ends of the coated fabric form the working electrode surfaces and are analogous in function to the cut distal end of the carbon fiber bundle described with respect to Figs. 1-5. For some applications, the back surface of the carrier film can be coated with silver silver chloride to provide a reference electrode. Alternatively, the reference electrode can comprise a separate skin-mounted electrode.

Wired enzyme sensors utilizing a fiber mesh substrate can have various medical applications. For example, if used as lactate sensors, sensors such as those shown in Figs. 10-13 can be used to determine perfusion levels in surgical procedures. For example, such sensors can be used in combination with products such as "surgical patties." Surgical patties are soft sterile textile pads that are positioned behind the retractor blades of surgical instruments. Such instruments, sometimes known as "spreaders", are used to hold overbearing tissue away from a surgeon's line of vision. Pressure exerted by the retractor blades against the tissue prevents adequate perfusion of the immediate contact area, often leading to cellular necrosis.

Fig. 14 schematically illustrates a sensing system configured for allowing a physician to monitor the lactate level in tissue that is being compressed by a retractor blade 400. A sensor 402 (e.g., a sensor configured similar to the sensors 120, 220 or 320 of Figs. 10-13) is positioned adjacent to the retractor blade 400. Preferably, the sensor comprises a wired enzyme sensor including a sensing material capable of oxidizing or reducing lactate. In one particular embodiment, the sensor 402 can include a redox compound and a redox enzyme that catalyzes an oxidation or reduction of lactate (e.g., lactate oxidase or lactate dehydrogenase). The sensor 402 is preferably mounted between the retractor blade 400 and a textile pad 404. In use, blood from the tissue being compressed by the retractor blade 400 defuses through the pad 404 to reach the sensor 402. A potential is preferably applied between a reference electrode 405 (e.g., a skin-mounted electrode) and the sensor 402. When the potential is applied, an electrical current will flow through the blood sample between the reference electrode 406 and the exposed working electrodes of the sensor 402. The current is the result of the electrolysis of lactate within the sample. This electrochemical reaction occurs via the redox compound in the sensing material at the working electrodes of the sensor 402 and the optional redox enzyme in the sensing material. A controller 408 is provided for measuring the current flow generated at a given potential. By measuring the current flow, the controller 408 can calculate a lactate concentration in the test sample. Consequently, by using the sensing system 401 in combination with the retractor blade 400, a surgeon can constantly monitor lactate level in the tissue being compressed by the retractor blade 400. If lactate levels begin to rise, the surgeon can remove the retractor blade 400 before the tissue is permanently damaged.

In the embodiments shown in Figs. 10-13, all of the fibers of the sensor can be electrically conductive. Alternatively, it may be desirable to alternate conductive and nonconductive fibers, or to provide specific regions of conductive fibers and other regions of nonconductive fibers. In still other embodiments, fibers oriented in one direction can be conductive, while fibers oriented in a perpendicular direction can be nonconductive. By varying the relative positioning of the conductive and nonconductive fibers, the operating characteristics of the sensors can be adjusted or otherwise modified.

With regard to the foregoing description, it is to be understood that changes may be made in detail, especially in matters of the construction materials employed and the shape, size and arrangement of the parts without departing from the scope of the present invention. Also, it should be noted that the sensors depicted in the drawings of this specification have been shown in a diagrammatic fashion and have not been drawn to scale. It is intended that the specification and depicted aspects be considered exemplary only, with a true scope of the invention being indicated by the broad meaning of the following claim.

## Claims

1. A sensor comprising :
a plurality electrically conductive fibers (22);
a dry sensing material (26) coating the outer surface of at least some of the fibers; and
an insulating layer (28) surrounding the plurality of electrically conductive fibers **characterized by** voids between the fibers that allow the analyte to diffuse into the sensor.

2. The sensor of claim 1, wherein the insulating layer forms an analyte barrier that surrounds the conductive fibers.

3. The sensor of claim 2, wherein the analyte barrier defines at least one opening for allowing an analyte to access the sensing material.

4. The sensor of claim 1, wherein the insulating layer comprises an electrical insulator.

5. The sensor of claim 1, wherein the insulating layer comprises polyurethane.

6. The sensor of claim 1, wherein the conductive fibers comprise carbon.

7. The sensor of claim 1, wherein the sensing material includes a redox compound.

8. The sensor of claim 7, wherein the redox compound comprises a transition metal complex with one or more organic ligands.

9. The sensor of claim 7, wherein the sensing material includes a redox enzyme.

10. The sensor of claim 9, wherein the redox enzyme catalyzes the oxidation or reduction of an analyte.

11. The sensor of claim 10, wherein the analyte comprises lactate.

12. The sensor of claim 11, wherein the redox enzyme is selected from the group of lactate oxidase and lactate dehydrogenase.

13. The sensor of claim 10, wherein the analyte comprises glucose.

14. The sensor of claim 13, wherein the redox enzyme is selected from the group of glucose oxidase and glucose dehydrogenase.

15. The sensor of claim 1, wherein the fibers form a sheet.

16. The sensor of claim 1, wherein the fibers are interwoven.

17. The sensor of claim 1, wherein the fibers form a piece of fabric.

18. The sensor of claim 2, wherein the analyte barrier defines a plurality of openings for allowing an analyte to access the sensing material.

19. A retractor device comprising:
a surgical retractor blade; and
a lactate sensor positioned adjacent to the retractor blade for sensing lactate levels in tissue being compressed by the retractor blade, the lactate sensor comprising:
a plurality of electrically conductive fibers (22);
a dry sensing material (26) coating at least the outer surface of some of the fibers, the dry sensing material including a redox compound for oxidizing or reducing lactate;
voids between the fibers that allow the analyte to diffuse into the sensor; and
an insulating layer (28) positioned about the plurality of electrically conductive fibers.

20. The retractor of claim 19, wherein the lactate sensor is mounted between said retractor blade and a surgical pad.

21. The retractor of claim 19, wherein the insulating layer defines a plurality of openings for allowing blood to access the sensing material on the fibers.

22. The retractor of claim 19, wherein the sensing material includes a redox enzyme that catalyzes the oxidation or reduction of lactate.

## Patentansprüche

1. Sensor, umfassend:
eine Vielzahl von elektrisch leitfähigen Fasern (22);
ein trockenes sensitives Material (26), das die Außenfläche zumindest einiger der Fasern ummantelt; und
eine Isolierschicht (28), welche die Vielzahl von elektrisch leitfähigen Fasern umgibt, **gekennzeichnet durch** Hohlräume zwischen den Fasern, die dem Analyt gestatten, in den Sensor zu diffundieren.

2. Sensor nach Anspruch 1, worin die Isolierschicht eine Analytschranke bildet, welche die leitfähigen Fasern umgibt.

3. Sensor nach Anspruch 2, worin die Analytschranke zumindest eine Öffnung definiert, um einem Analyt zu gestatten, zu dem sensitiven Material zu gelangen.

4. Sensor nach Anspruch 1, worin die Isolierschicht einen elektrischen Isolator umfasst.

5. Sensor nach Anspruch 1, worin die Isolierschicht Polyurethan umfasst.

6. Sensor nach Anspruch 1, worin die leitfähigen Fasern Kohlenstoff umfassen.

7. Sensor nach Anspruch 1, worin das sensitive Material eine Redoxverbindung enthält.

8. Sensor nach Anspruch 7, worin die Redoxverbindung einen Übergangsmetallkomplex mit einem oder mehreren organischen Liganden umfasst.

9. Sensor nach Anspruch 7, worin das sensitive Material ein Redoxenzym enthält.

10. Sensor nach Anspruch 9, worin das Redoxenzym die Oxidation oder Reduktion eines Analyts katalysiert.

11. Sensor nach Anspruch 10, worin der Analyt Laktat umfasst.

12. Sensor nach Anspruch 11, worin das Redoxenzym aus der Gruppe Laktatoxidase und Laktatdehydrogenase ausgewählt ist.

13. Sensor nach Anspruch 10, worin der Analyt Glukose umfasst.

14. Sensor nach Anspruch 13, worin das Redoxenzym aus der Gruppe Glukoseoxidase und Glukosedehydrogenase ausgewählt ist.

15. Sensor nach Anspruch 1, worin die Fasern ein Blatt bilden.

16. Sensor nach Anspruch 1, worin die Fasern miteinander verwoben sind.

17. Sensor nach Anspruch 1, worin die Fasern ein Stück Gewebe bilden.

18. Sensor nach Anspruch 2, worin die Analytschranke eine Vielzahl von Öffnungen definiert, um einem Analyt zu erlauben, zu dem sensitiven Material zu gelangen.

19. Retraktoreinrichtung, umfassend:
eine chirurgische Retraktorklinge; und
einen Laktatsensor, der benachbart zu der Retraktorklinge angeordnet ist, zur Erfassung von Laktatspiegeln in Gewebe, das durch die Retraktorklinge komprimiert wird, wobei der Laktatsensor umfasst:
eine Vielzahl von elektrisch leitfähigen Fasern (22);
ein trockenes sensitives Material (26), das zumindest die Außenfläche einiger der Fasern ummantelt, wobei das trockene sensitive Material eine Redoxverbindung zum Oxidieren oder Reduzieren von Laktat enthält;
Hohlräume zwischen den Fasern, die dem Analyt gestatten, in den Sensor zu diffundieren, und
eine Isolierschicht (28), die um die Vielzahl von elektrisch leitfähigen Fasern herum angeordnet ist.

20. Retraktor nach Anspruch 19, worin der Laktatsensor zwischen der Retraktorklinge und einem chirurgischen Tampon montiert ist.

21. Retraktor nach Anspruch 19, worin die Isolierschicht eine Vielzahl von Öffnungen definiert, um Blut zu erlauben, zu dem sensitiven Material auf den Fasern zu gelangen.

22. Retraktor nach Anspruch 19, worin das sensitive Material ein Redoxenzym enthält, das die Oxidation oder Reduktion von Laktat katalysiert.

## Revendications

1. Détecteur comprenant :
une pluralité de fibres électriquement conductrices (22) ;
un matériau de détection à sec (26) recouvrant la surface externe d'au moins certaines des fibres ; et
une couche isolante (28) entourant la pluralité de fibres électriquement conductrices, **caractérisé par** des vides situés entre les fibres qui permettent à l'analyte de diffuser dans le détecteur.

2. Détecteur selon la revendication 1, dans lequel la couche isolante forme une barrière contre les analytes qui entoure les fibres conductrices.

3. Détecteur selon la revendication 2, dans lequel la barrière contre les analytes délimite au moins une ouverture permettant à un analyte de parvenir jusqu'au matériau de détection.

4. Détecteur selon la revendication 1, dans lequel la couche isolante comprend un isolant électrique.

5. Détecteur selon la revendication 1, dans lequel la couche isolante comprend du polyuréthane.

6. Détecteur selon la revendication 1, dans lequel les fibres conductrices comprennent du carbone.

7. Détecteur selon la revendication 1, dans lequel le matériau de détection comprend un composé redox.

8. Détecteur selon la revendication 7, dans lequel le composé redox comprend un complexe associant un métal de transition et un ou plusieurs ligands organiques.

9. Détecteur selon la revendication 7, dans lequel le matériau de détection comprend une enzyme redox.

10. Détecteur selon la revendication 9, dans lequel l'enzyme redox catalyse l'oxydation ou la réduction d'un analyte.

11. Détecteur selon la revendication 10, dans lequel l'analyte comprend du lactate.

12. Détecteur selon la revendication 11, dans lequel l'enzyme redox est choisie dans le groupe constitué de la lactate oxydase et de la lactate déshydrogénase.

13. Détecteur selon la revendication 10, dans lequel l'analyte comprend du glucose.

14. Détecteur selon la revendication 13, dans lequel l'enzyme redox est choisie dans le groupe constitué de la glucose oxydase et de la glucose déshydrogénase.

15. Détecteur selon la revendication 1, dans lequel les fibres forment une feuille.

16. Détecteur selon la revendication 1, dans lequel les fibres sont entrelacées.

17. Détecteur selon la revendication 1, dans lequel les fibres forment un morceau de tissu.

18. Détecteur selon la revendication 2, dans lequel la barrière contre les analytes délimite une pluralité d'ouvertures permettant à l'analyte de parvenir jusqu'au matériau de détection.

19. Dispositif rétracteur comprenant :
une lame de rétracteur chirurgical ; et
un détecteur de lactate positionné à proximité de la lame du rétracteur pour détecter les niveaux de lactate présents dans les tissus comprimés par la lame du rétracteur, le détecteur de lactate comprenant :
une pluralité de fibres électriquement conductrices (22) ;
un matériau de détection à sec (26) recouvrant au moins la surface externe de certaines des fibres, le matériau de détection à sec comprenant un composé redox destiné à oxyder ou réduire le lactate ;
des vides situés entre les fibres pour permettre à l'analyte de diffuser dans le détecteur ; et
une couche isolante (28) positionnée autour de la pluralité de fibres électriquement conductrices.

20. Rétracteur selon la revendication 19, dans lequel le détecteur de lactate est monté entre ladite lame du rétracteur et une compresse chirurgicale.

21. Rétracteur selon la revendication 19, dans lequel la couche isolante délimite une pluralité d'ouvertures permettant au sang de parvenir jusqu'au matériau de détection présent sur les fibres.

22. Rétracteur selon la revendication 19, dans lequel le matériau de détection comprend une enzyme redox qui catalyse l'oxydation ou la réduction du lactate.
